# EUROPEAN PATENT SPECIFICATION

(11) **EP 0 205 236 B1**
(45) Date of publication and mention of the grant of the patent: **20.01.1993**
(21) Application number: 86302650.6
(22) Date of filing: 10.04.1986
(51) Int. Cl.: G01N 21/75, G01N 33/553

(54) **Improvements in or relating to Optic-Waveguide Biosensors**
Messfühler für biologische Moleküle mit Verwendung optischer Wellenleiter
Capteurs biologiques à guides d'ondes optiques

(30) Priority: 12.04.1985 GB 8509491
(43) Date of publication of application: 17.12.1986
(73) Proprietor: GEC-MARCONI LIMITED, Stanmore, Middlesex HA7 4LY (GB)
(72) Inventor: Stewart, William James, Blakesley Northants, NN12 8RE (GB)
(74) Representative: Pope, Michael Bertram Wingate

(56) References cited:
- ANALYTICAL CHEMISTRY, vol. 54, no. 9, August 1982, pages 1071(A)-1080(A), American Chemical Society; I. CHABAY: "Optical waveguides"
- CLINICAL CHEMISTRY, vol. 30, no. 9, September 1984, pages 1533-1538, Winston-Salem, NC, US; R.M. SUTHERLAND et al.: "Optical detection of antibody-antigen reactions at a glass-liguid interface"
- APPLIED OPTICS, vol. 21, no. 12, June 1982, pages 2167-2173, Optical Society of America, New York, US; B. BOSACCHI et al.: "Resonant frustrated-total-reflection technique for the characterization of thin films"
- SENSORS AND ACTUATORS, vol. 4, 1983, pages 299-304, Elsevier Sequoia, Amsterdam, NL; B. LIEDBERG et al.: "Surface plasmon resonance for gas detection and biosensing"

## Description

The present invention concerns optic-waveguide biosensors i.e. sensors for detecting and/or monitoring or quantifying the presence and/or behaviour of specific assay molecular species in test fluid samples. The invention has application to, for example: immunoassay (the detection of antibodies, antigens, or hormones in blood samples), pollution monitoring; and monitoring of clinical diagnostic reactions involving e.g. enzymes and the like.

In an article entitled "Detection of Antibody - Antigen Reactions at a glass-liquid Interface as a Novel Optical Immunoassay Concept", (1984) (Proceedings of 2nd Optical Fibre Conference (Stuttgart 1984) page 75), R.M. Sutherland et al describe a biosensor wherein an antibody species is covalently immobilized onto the surface of a planar or fibre-optic waveguide. The reaction of immobilized antibody with antigen in sample solution is detected using the evanescent wave component of a light beam, totally internally reflected many times within the waveguide. The evanescent wave has a characteristic penetration depth of a fraction of a wavelength into the aqueous phase, thus optically interacting with substances bound to or very close to the interface and only minimally with the bulk solution.

Reference is also made to United Kingdom Patent Application GB-A-2156970, which discloses optic-waveguide biosensors and a similar technique.

In 'Analytical Chemistry', vol. 54, no.9, August 1982, pages 1071-1080, a technique for analysis of a film coating is described in which light is coupled into and out of an optical body, on which the coating is formed, via prisms.

The present invention is intended to enhance the device response for a given beam power.

In accordance with the invention there is provided an optic-waveguide biosensor comprising a waveguide having an interface with a coating sensitized for a given assay species; said waveguide having a higher refractive index than said coating, and two light coupling members adjacent to the waveguide, to direct light into and out of said waveguide respectively, characterised in that said biosensor includes a solid dielectric medium interposed between the waveguide and one or both of the said coupling members and having a light reflecting and partially transmissive interface with said waveguide and an interface with said one or both coupling members; said waveguide having a refractive index relative to said medium such that said waveguide and said interfaces of said waveguide with said coating and with said medium together constitute a mirrored resonant cavity.

The power of radiation within the cavity is thus enhanced relative to the power of the input beam and the power of the interactive evanescent wave extending into the sensitive coating is likewise enhanced, thereby improving device sensitivity to species absorbed by the coating.

The solid dielectric medium may be a thin single layer of transparent material. Alternatively, it may be a dielectric multilayer structure.

In the accompanying drawings:-
Figure 1 is an illustrative cross-section view of a known optic-waveguide biosensor;
Figure 2 is an illustrative cross-section view of an optic-waveguide biosensor modified in accordance with this invention; and,
Figure 3 illustrates schematic refractive index profiles for a modified bionsensor incorporating
   a) a single layer reflector; and,
   b) a multi-layer structure reflector, respectively.

Embodiments of the invention will now be described, by way of example only, with reference to the drawings accompanying this specification.

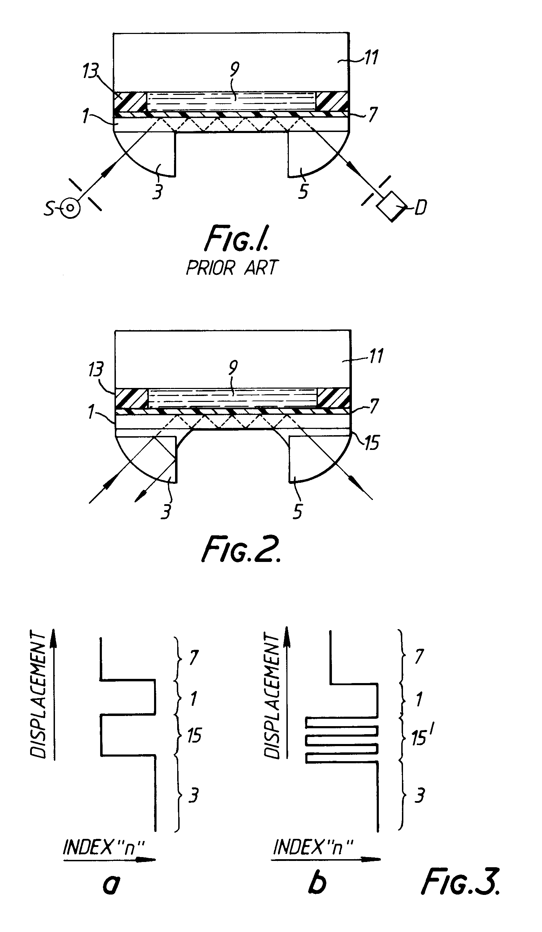

In Figure 1 there is shown the form of optic waveguide biosensor disclosed in the previously mentioned article by R.M. Sutherland et al in which light from a source S is directed into a planar waveguide 1 by means of a first coupling prism 3 and propagated by multiple total internal reflection to exit by means of a second coupling prism 5 whence it is directed onto a light detector D. The external surface of the waveguide 1 is provided with a sensitized organic coating 7. The latter is exposed to a sample liquid 9 which is contained by means of a flow cell 11 and gasket 13 arrangement. In the coating 7, antibody material is covalently immobilized and this responds to any specific antigen material in the sample liquid to which it is exposed. The waveguide is of fused quartz material and this provides a large differential in optical density between the quartz waveguide 1 (high refractive index n₁) and the adjacent coating 7 (low refractive index n₂). Light is totally internally reflected within the body of the waveguide 1, a portion of the optical power, however, propagating as an evanescent wave in the coating medium 7. The binding of antigen by the immobilised antibody is monitored by a resultant increase in the light absorption measured at the detector D.

In the inventive modification shown in Figure 2, a layer 15 (for example an evaporated or sputtered layer of magnesium fluoride) is interposed between the coupling prisms 3, 5 and the planar waveguide 1. The layer 15 has a lower refractive index than the waveguide 1 and the interface between the layer 15 and waveguide 1 is light reflecting and partially transmissive. This construction may be used in conjunction with an infra-red light injection laser as light source S having typical light wavelength of 0.8 µm. Similar apparatus may be used for visible light and for ultra-violet light, but in the latter case a layer 15 of alumina or similar material would be used where the typical light wavelength is 270nm. Light is coupled to the waveguide 1 by frustrated total internal reflection, and the thickness of the interposed layer 15 is chosen accordingly. The refractive index profile for this assembly of media - coating 7, waveguide 1, layer 15 and coupling member 3 - is shown in Figure 3(a). As can be seen, the waveguide 1 is isolated by media 7, 15 of lower refractive index. Incident light coupled into the waveguide is thus resonantly trapped in the mirrored cavity between the interfaces of the waveguide 1 with the layer 15 and the coating 7. The power level is high in this cavity region. The guided light subsequently leaks back into the bulk medium, the second coupling member 5, after which it is monitored by the photodetector D. The evanescent wave in the coating 7 will interact with any absorbed species in this coating 7 and in turn therefore will modify the absorbtion and phase shift of the light beam monitored The latter is enhanced by this resonant effect.

As an alternative modification, the single interposed layer 15 of the assembly shown in Figure 2 may be replaced by a dielectric multilayer structure 15'. A typical index profile for this modified assembly is shown in Figure 3(b). As an example, a multilayer structure providing 90% reflection 10% transmission provided a factor of x10 enhancement of power within the resonant cavity.

The presence of assay species may be detected and/or monitored by measuring changes in the absorbtion or polarisation of the monitored light beam. The interaction will depend on the frequency and angle of incidence of the light beam. Thus source S and detector D may be singular components mechanically scanned over a range of angles, or may each comprise an extended array, each component being electronically addressed to simulate a scan. Alternatively, the source S and detector D may be set up in an optimal static configuration.

## Claims

1. An optic-waveguide biosensor comprising:- a waveguide (1) having an interface with a coating (7) sensitized for a given assay species, said waveguide (1) having a higher refractive index than said coating (7) and two light coupling members (3, 5) adjacent to the waveguide (1), to direct light into and out of said waveguide (1) respectively, characterised in that said biosensor includes a solid dielectric medium (15, 15') interposed between the waveguide (1) and one or both of said coupling members (3, 5) and having a light reflecting and partially transmissive interface with said waveguide (1) and an interface with said one or both coupling members (3, 5); said waveguide (1) having a refractive index relative to said medium (15, 15') such that said waveguide and said interfaces of said waveguide (1) with said coating (7) and with said medium (15, 15') together constitute a mirrored resonant cavity.

2. A biosensor, as claimed in Claim 1, wherein the solid dielectric medium comprises a single layer (15) of material and is of thickness such as to afford light coupling by frustrated total internal reflection with said waveguide (1).

3. A biosensor, as claimed in Claim 2, wherein the single layer (15) is of magnesium fluoride material.

4. A biosensor, as claimed in Claim 2, wherein the single layer (15) is of alumina material.

5. A biosensor, as claimed in Claim 1, wherein the solid dielectric medium comprises a dielectric multilayer structure (15').

6. A biosensor, as claimed in Claim 5, wherein the multilayer structure (15') provides 90% reflection 10% transmission.

## Patentansprüche

1. Meßfühler für biologische Moleküle mit Verwendung optischer Wellenleiter, aufweisend einen Wellenleiter (1), der eine Grenzfläche mit einer Beschichtung (7) aufweist, die für eine gegebene Prüfgattung sensibilisiert ist, wobei der Wellenleiter (1) einen höheren Brechungsindex als die Beschichtung (7) aufweist, und zwei an den Wellenleiter angrenzende Lichtkopplungsteile (3,5), die Licht in den Wellenleiter (1) hinein- bzw. herausleiten, dadurch gekennzeichnet, daß der Biomeßfühler ein dielektrisches Festkörpermedium (15,15') umfaßt, das zwischen dem Wellenleiter (1) und einem oder beiden Kopplungsteilen (3,5) angeordnet ist und eine lichtreflektierende und teilweise durchlässige Grerzfläche mit dem Wellenleiter (1) und eine Grenzfläche mit einem oder beiden Kopplungsteilen (3,5) aufweist; daß der Wellenleiter (1) einen derartigen Brechungsindex in Bezug auf dieses Medium (15,15') aufweist, daß der Wellenleiter und die Grenzflächen des Wellenleiters (1) mit der Beschichtung (7) und mit diesem Medium (15,15') gemeinsam einen verspiegelten Resonanzhohlraum bilden.

2. Biomeßfühler nach Anspruch 1, in welchem das dielektrische Festkörpermedium eine einzelne Schicht (15) aus solchem Material und solcher Dicke umfaßt, daß eine Lichtkopplung durch behinderte interne Totalreflexion mit dem Wellenleiter (1) zustande kommt.

3. Biomeßfühler nach Anspruch 2, in welchem die einzelne Schicht (15) aus Magnesiumfluoridmaterial besteht.

4. Biomeßfühler nach Anspruch 2, in welchem die einzelne Schicht (15) aus Aluminiumoxidmaterial besteht.

5. Biomeßfühler nach Anspruch 1, in welchem das dielektrische Festkörpermedium eine dielektrische mehrschichtige Struktur (15') umfaßt.

6. Biomeßfühler nach Anspruch 5, in welchem die mehrschichtige Struktur (15' ) 90% Reflexion und 10% Transmission liefert.

## Revendications

1. Biocapteur à guide d'onde optique, comprenant un guide d'onde (1) ayant une interface avec un revêtement (7) sensibilisé à une espèce donnée analysée, le guide d'onde (1) ayant un indice de réfraction supérieur à celui du revêtement (7), et deux organes (3, 5) de couplage de lumière, adjacents au guide d'onde (1) et destinés à diriger la lumière dans le guide d'onde (1) et à l'extraire de celui-ci, caractérisé en ce que le biocapteur comporte une matière diélectrique solide (15, 15') disposée entre le guide d'onde (1) et l'un des organes de couplage (3, 5) ou les deux, et formant une interface de réflexion de lumière qui transmet partiellement avec e guide d'onde (1), et une interface avec le premier organe de couplage (3, 5) ou les deux, le guide d'onde (1) ayant un indice de réfraction, par rapport à la matière (15, 15'), qui est tel que le guide d'onde et les interfaces du guide d'onde (1) avec le revêtement (7) et avec la matière (15, 15') constituent ensemble une cavité résonante à miroirs.

2. Biocapteur selon la revendication 1, dans lequel la matière diélectrique solide comporte une seule couche (15) de matière et a une épaisseur telle qu'elle assure le couplage de lumière par réflexion interne totale frustrée avec le guide d'onde (1).

3. Biocapteur selon la revendication 2, dans lequel la couche unique (15) est formée de fluorure de magnésium.

4. Biocapteur selon la revendication 2, dans lequel la couche unique (15) est formée d'alumine.

5. Biocapteur selon la revendication 1, dans lequel la matière diélectrique solide est une structure diélectrique multicouche (15').

6. Biocapteur selon la revendication 5, dans lequel la structure multicouche (15') donne 90 % de réflexion et 10 % de transmission.
